# EUROPEAN PATENT SPECIFICATION

(11) **EP 2 167 156 B1**
(45) Date of publication and mention of the grant of the patent: **24.01.2018**
(21) Application number: 08762724.6
(22) Date of filing: 27.05.2008
(51) Int. Cl.: A61M 1/00

(54) **MEDICAL APPARATUS**
MEDIZINISCHES GERÄT
APPAREIL MÉDICAL

(30) Priority: 01.06.2007 IT MO20070189
(43) Date of publication of application: 31.03.2010
(73) Proprietor: Rand S.r.l., 41036 Medolla (MO) (IT)
(72) Inventor: GALAVOTTI, Daniele, 41037 Mirandola (MO) (IT)
(74) Representative: Bergamini, Silvio
(86) International application number: PCT/IB2008/001325
(87) International publication number: WO 2008/146132

(56) References cited:
- WO-A-2004/084792
- WO-A-2006/041406
- US-A- 4 466 888
- US-A- 4 898 572

## Description

### TECHNICAL FIELD.

The invention relates to a medical apparatus, especially suitable for filtration and accumulation of solutions for intra-abdominal and/or intrapleuric perfusion in a hyperthermal regime.

### BACKGROUND ART.

The medical treatment of many serious pathologies, such as e.g peritoneal and pleuric neoplasias, require the creation of extra-corporeal circuits which are destined to be passed-through by perfusion solutions after surgical operations to reduce the neoplasias.

In these circuits a device must be provided which has the specific function of filtering, accumulating and managing variable volumes of solutions which are perfused in the abdominal and/or pleuric cavity of a patient.

At present there are no available apparatus which are specifically realised for this type of therapy and, for this reason, devices made originally for use in other treatments are used, such as, e.g., heart surgery treatments.

These devices essentially comprise a filtering element which is destined to filter the blood of a patient during a surgical operation, in order to remove all those impurities deriving from the operation, such as, for example, clots, fragments of cut tissues, fibrin, gelatinous tissues, suture stitches and residues thereof.

Further, these devices are of the so called "open" type, i.e. they exhibit at least an opening which places the internal environment of the devices in contact with the outside environment.

An "Autotrasfuser device" is known from the US patent No.: 4,898,572 according to which it is known removing the blood from the patient and returning the blood to the patient.

The device includes a collection bag suspended in an air jacket. A valve is provided in the device to selectively apply a negative pressure to the air jacket and the interior of the collection bag to draw the blood from the patient. The valve is subsequently rotated to apply a positive pressure to the air jacket and the outside of the bag to compress the bah and cause the blood to be returned to the patient. A plurality of filter members are provided in the collection chamber to remove blood clots and undesirable solids.

According to international patent application WO2004/084729 A1 a "Device for a body fluid bag" is also known.

The device comprise a filtering part and a re-transfusion part, the filtering part having a filtering part inlet opening and a filtering part outlet opening. The outlet opening is arranged to be connected to an inlet opening of said body fluid bag, and the re-transfusion part has re-transfusion part inlet opening arranged to be connected to an outlet opening of said body fluid bag and an re-transfusion part outlet opening. The filtering part and the re-transfusion part are mechanically integrated to form a single unit.

According to the international patent application WO2006/041406 a "Method and apparatus for auto-transfusion" is also known.

The apparatus comprises a first chamber for collecting blood through an inlet port; a second chamber for receiving blood from the first chamber through a connection channel and a channel valve manually controllable to close the convection channel.

Further the apparatus comprises a control means for simultaneously controlling the channel valve and the internal pressure of second chamber. Still further, a lipid separator is arranged in the second chamber for separating the blood from lipids before the realise trough the realise port.

According to the US patent No.: 4,466,888 a "Suction liquid collection assembly and flexible collecting bag therefor" are known.

The blood collecting bag s clamped along its peripheral edges between two shells. Inlet and outlet ports are formed in one side edge of the bag and are also clamped between the shells. The shells form a vacuum chamber around the bag with the bag serving as a sealing gasket and also house a liquid trap connected to the gas outlet from the bag. Pressure between the two shells is equalized through a vent hole formed through the bag. The bag comprises a membrane forming front and rear faces joined along peripheral edges. The disposable bag includes a laminated filter which lies flat between the front and the rear faces of the bag when the bag is collapsed. The filter is sealed between the two faces of the bag along one edge thereof and is joined to the rear face of the bag along its opposite edge to define inlet and outlet chambers. Preferably, the shells are of clear plastic moulded to conform to the shape of the expanded bag within the chamber.

The above-described devices exhibit some drawbacks.

A first drawback is that the devices tend to rapidly saturate the filtering elements they are made of, which thus quickly become useless for their allotted function.

A further drawback is that being of the open type, damaging gases developing with the use of chemotherapeutical drugs, used at temperatures of above 37°C, might be diffused into the environment.

A further drawback is that these devices are realised using plastic materials which can react chemically with chemotherapeutical drugs and release very damaging residues of these plastic materials into the solutions perfused into the patient.

A further drawback is that the accumulation capacity of these known devices is considerably limited in relation to use thereof in surgical treatment of peritoneal and pleuric neoplasias.

During surgical operations, the patient has to be perfused with volumes of solution which vary between a minimum of 2,000 ml up to a maximum of 6,000 to 9,000 ml, and these capacities are not reached in the known devices normally used in heart surgery and used in the treatment of peritoneal or pleuric neoplasias.

In perfusion, volumes of solutions used are perfused into the patient's abdomen or pleura, by means of a suitable perfusion circuit which has an outgoing branch and a return branch which flow into or out of the previously-mentioned devices for filtering these volumes of solution before they are newly perfused in the patient's abdomen or pleura, after being heated to a predetermined temperature.

### OBJECTS OF THE INVENTION.

An object of the invention is to provide an improvement over the prior art.

A further object of the invention is to realise a medical apparatus which can be used specifically in surgical treatments in hyperthermal regime on peritoneal and pleuric neoplasias, without causing any risk of release of damaging substances.

A further object of the invention is to realise a medical apparatus which does not release gases into the atmosphere which might be damaging to the health of the operators or patients.

A further object of the invention is to realise a medical apparatus which has sufficient capacity to contain all the volumes of solutions used during surgical operations to reduce neoplasias, such as, e.g., peritoneal and/or pleuric neoplasias.

A further object of the invention is to realise a medical apparatus which can perform an efficient filtration action on the solutions to be infused into a patient, in order to prevent the patient from coming into contact with substances that have previously been removed from the operating area.

According to an aspect of the invention a medical apparatus is provided which is particularly suitable for filtration and accumulation of solutions for intra-abdominal and/or intrapleuric perfusion in a hyperthermal regime, comprising a body, said body comprising: inlet and outlet means for liquid solutions to be filtered; filtering means arranged between said inlet and outlet means; characterized in that said filtering means are arranged in a receiving element of said liquid solutions, a container element of said liquid solutions being provided, which container element having a variable capacity and being connected to said receiving element and to said outlet means.

The medical apparatus enables receiving, containing, filtering and newly perfusing a patient with liquid solutions used during surgical operations, for intra-abdominal and/or intrapleural perfusion in a hyperthermal regime, while also preventing diffusion into the outside environment of dangerous vapours generated by drugs, in particular chemotherapeutical drugs.

### BRIEF DESCRIPTION OF THE DRAWINGS

Further characteristics and advantages of the invention will better emerge from the description that follows of a medical apparatus, illustrated by way of non-limiting example in the accompanying figures of the drawings, in which:
figure 1 is a schematic view of an accumulation, filtration and perfusion system of liquid medical solutions to patients, in a stage of preparation for an infusion;

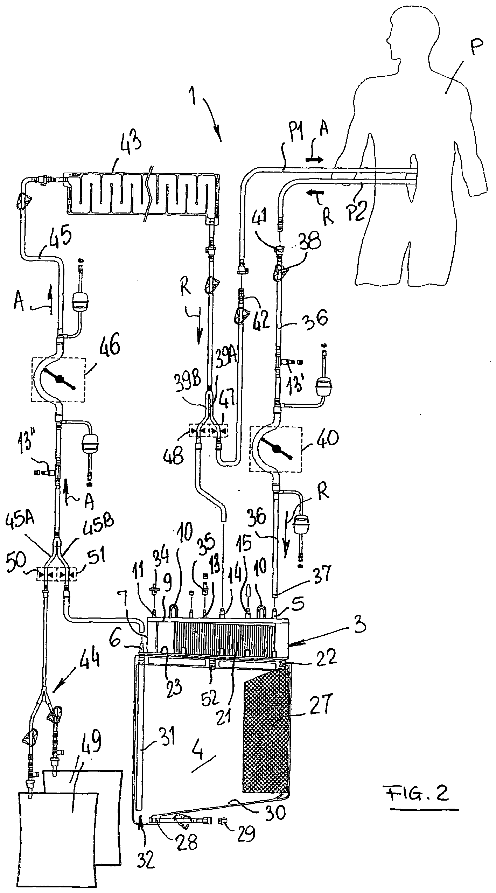

figure 2 is a schematic view of the system of figure 1, in a stage of perfusion of a liquid solution to a patient;
figure 3 is a perspective exploded view of a medical apparatus which is part of the system of figure 1;

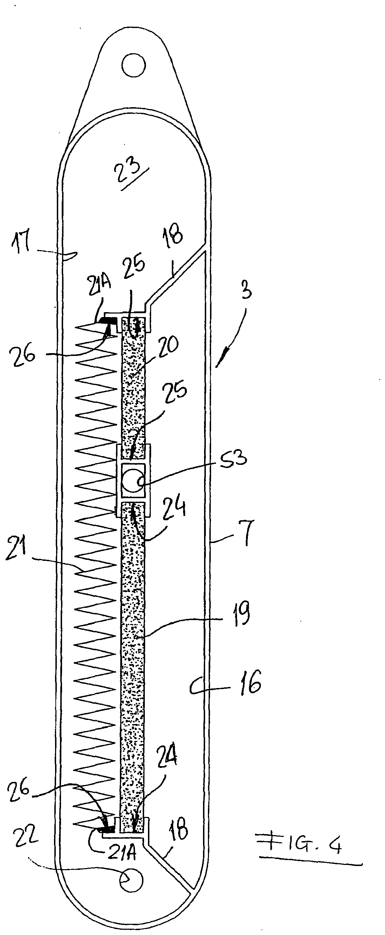

figure 4 is a view in enlarged scale of a transversal section of the medical apparatus of figure 3, taken along a plane III-III of figure 3.

With reference to the figures of the drawings, 1 denotes a pre-assembled system, known as a set and called such hereinafter, for intra-abdominal and/or intrapleuric perfusion in a hyperthermal regime of medical liquid solutions.

The set 1 comprises a medical apparatus 2 suitable for accumulating and filtering medical liquid solutions which circulate in the set 1, as described herein below.

The medical apparatus 2 comprises a substantially-rigid receiving element 3, to a lower surface of which a flexible bag 4 is removably and sealedly fixed, which bag 4 is destined to contain volumes of medical liquid solutions.

In the receiving element 3 are defined an inlet 5 and an outlet 6, both provided with a closing lid 9.

Apart from the inlet 5, the closing lid 9 exhibits two hooks 10 destined to enable the medical apparatus 2 to be hung from two corresponding supports of a weighing device of an automatic machine used for perfusing medical solutions in patients P.

As well as the hooks 10 and the inlet 5, the closing lid 9 further exhibits other holes, precisely a compensating hole 11 for compensating pressure differences between the inside of the bag 4 and the external environment, a supplementary inlet hole 12 for use if it should become necessary to introduce solutions or drugs into the bag 4, an introduction hole 13 for introducing chemotherapy drugs or drugs in general, a recirculating hole 14, a second supplementary hole 15, normally closed but openable if necessary.

All of these holes are provided, like the inlet 5 and the outlet 6, with respective mouths, normally frustum-conical, to facilitate insertion and attachment of corresponding tube ends, as will be explained in more detail herein below.

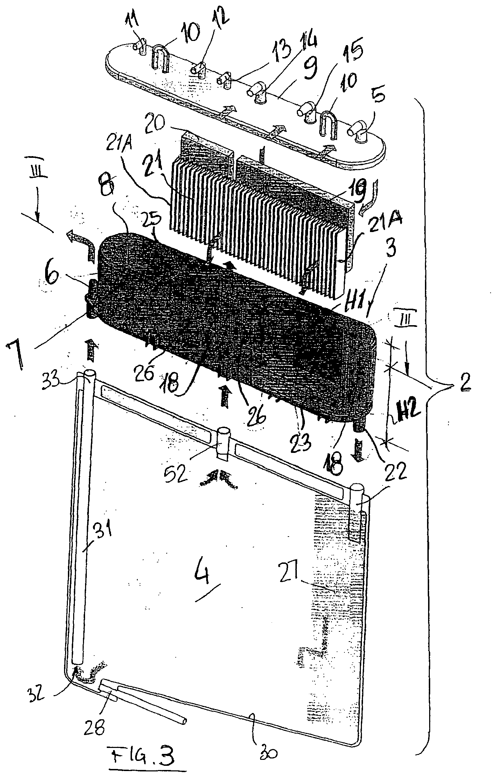

With reference to figure 3, the box structure 7 exhibits the internally defined compartment which is sub-divided into two chambers, respectively a receiving chamber 16 and a conveying chamber 17, substantially separated from one another by a wall 18 which is arranged longitudinally internally of the box structure 7.

Note that all of the holes, from 12 to 15, as well as the inlet 5, which are substantially aligned to one another, open above the receiving chamber 16.

Further, the height H1 of the wall 18 is slightly lower than the height H2 of the box structure 7, such that it leaves an emergency passage opening defined between the upper edge of the wall 18 and the lower surface of the lid 9 when the lid 9 is mounted on the receiving element 7.

The wall 18 shapes housing seats 24, 25, 26 for housing a series of flat filters 19, 20, 21 which are all of a substantially equal height to the height H1 of the wall 18, such liquid solutions is in any case possible through the emergency passage opening defined between the upper edge of the wall 18 and the lower surface of the lid 9. Precisely, the filters 19 and 20 have substantially the same filtration coefficient while the filter 21 has a filtration coefficient which is considerably higher than that of filters 19 and 20, such as to filter the liquid solutions passing from the receiving chamber 16 to the conveying chamber 17 through the filters in a more capillary way.

For this reason, the wall 18 exhibits, at the seats 24, 25, 26, passages or through-openings which force the liquid substances to cross the filters 19, 20, 21.

In particular, the base of the filter 21 resting on the bottom 23 is glued to the bottom 23, and the sides 21A are glued to the lateral sides defining the seat 26 in which the filter 21 is arranged.

A discharge opening 22 is afforded in the conveying chamber 17, and more precisely on the bottom 23 thereof, which is also the bottom of the receiving chamber 16.

The bottom 23 is slightly inclined towards this discharge opening 22 such as to convey the liquid solutions towards the opening 22 after they have been filtered by the filters 19,20,21.

The opening 23 opens into the bag 4 in proximity of a side thereof, such that the liquid solutions which pass through are forced to pass through a further filter 27 which is arranged inside the bag 4, close to a side thereof and extending substantially down to the bottom 30 of the bag 4, which bottom 30 is also inclined, where there is a discharge opening 28 closed with a cap 29 which can be removed when necessary.

A tube 31 is arranged internally of the bag 4 and in proximity of an opposite side to the side where the further filter 27 is arranged, which tube 31 has an end facing towards the bottom 30 and located close thereto, and an opposite end 33 which is connected to the outlet 6.

The compensating hole 11, which is normally kept open, is provided with a filter 34 for protecting the contents of the bag 4 from the bacteria which are found in the outside environment.

This filter 34 comprises an internal filtering membrane coupled to a coal insert: the first membrane prevents bacteria access to the inside of the bag 4, while the coal insert retains any vapours from chemotherapy drugs which tend to exit towards the external environment when a greater pressure than atmospheric pressure is generated inside the bag 4.

The introduction of hole 14 is provided with an "injection point" 35, i.e. a shutter member made of a plastic material, e.g. a unidirectional valve, which can be activated by introducing the attachment mouth of the needle of a syringe in order to introduce drugs internally of the receiving element 7 and thus into the bag 4.

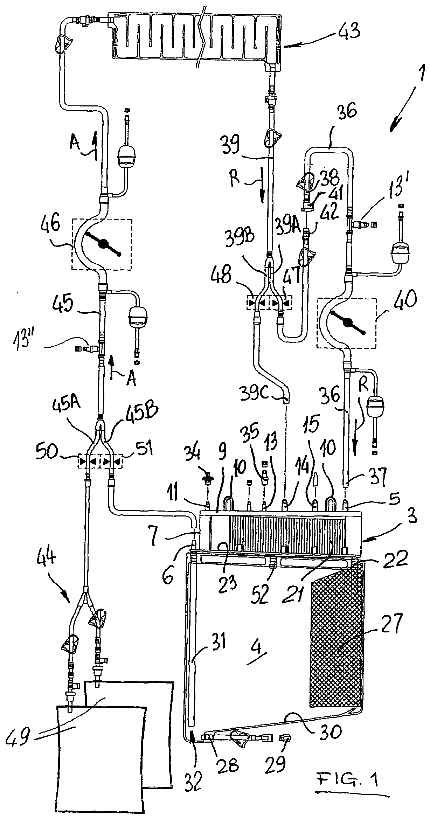

With reference to figure 1, the medical apparatus 2 is connected to a series of tubes which connect it with the patient P via a pumping machine.

The tubes comprise a first tube 36 which is predisposed to be connected at an end 37 thereof to the inlet 5 and at the opposite end thereof to an exit P2 from the body of the patient P subjected to a surgical operation, with the interposing of a second tube 39, as is better explained herein below.

A first pump 40 is located along the first tube 36, normally a peristaltic pump, which creates an aspiration internally thereof.

The opposite end 38 is provided with a connector 41, e.g. a female connector, which is couplable with a connector 42, e.g. a male connector, which is mounted at a terminal end of a branch 39A of the second tube 39, which has a further branch 39B the end 39C of which is connectable to the recirculating hole 14; clamps 47 and 48, openable and closable according to need, are arranged on both branches 39A and 39B. The second tube 39 is connected to an outlet line from the patient P, with the interposing of a heating device 43 which heats the solutions to be perfused to a temperature which is substantially higher than body temperature, i.e. beyond 37°C. The set 1 further comprises a third tube 45 which is destined to send the liquid solutions to be perfused to the patient P through an inlet P1 connected to the patient, as indicated by arrows A.

A second pump 46 is provided along the third tube 45, which second pump 46 aspirates the solutions to be perfused from the bag 4 when the set 1 is at normal functioning regime or, at the start of the perfusion cycle, from a further bag 49 in which a solution to be perfused is contained and kept.

In more detail, the third tube 45 has two branches 45A and 45B which flow into the tube 45 upstream of the second pump 46.

Further clamps 50 and 51 are mounted on both branches 45A and 45B, which clamps 50 and 51 can be opened or closed according to need.

In a practical realisation, both the openable clamps 47 and 48 and the further clamps 50 and 51 can be part of the overall equipment of an automatic machine for perfusing liquid solutions on which the set 1 is mounted for this purpose.

In more detail, the branch 45B is connected to the outlet 6 while the branch 45A is connected to the further bag 49.

The bag 4 also has a central breather opening 52 which is connected to a corresponding opening 53 which is obtained in the bottom 23 of the receiving element 7, destined to expel through the receiving element 7 and the compensating hole 11 any air bubbles which may be present in the bag 4.

The functioning of the medical apparatus 2 comprises three main stages, i.e. a first preparation stage, a second treatment stage and a third emptying stage.

In turn, the first preparation stage comprises a filling stage and a recirculation stage. With reference to figure 1, during the filling stage the further clamp 50 is in an open configuration while the further clamp 51 is in a closed configuration.

The clamp 47 is in a closed configuration while the clamp 48 is in the open configuration.

The male connector 42 of the branch 39A is fastened on the female connector 41 of the first tube 36.

In these conditions the liquid solution contained in the further bag 49 is aspirated internally of the set 1 by the second pump 46 which sends it to the heating device 43 and from there to inside the medical apparatus 2, through the branch 39B of the second tube.

This stage serves to fill the whole set 1 with the quantity of liquid solution necessary for the perfusion and predetermined on the basis of the built of the patient P.

During the recycling stage, still with reference to figure 1, the further clamp 50 is closed while the further clamp 51 is opened.

The clamp 47 is opened while the clamp 48 is closed.

The male connector 42 of the branch 39A is still connected to the female connector 41 of the first tube 36.

In these conditions, the liquid solution contained in the bag is aspirated by the second pump 46 through the internal tube 31 and the outlet 6 and is sent towards the heating device 43 where it is heated to a temperature of about 40°C.

The liquid solution is sent from the heating device 43 towards the first pump 40 which via the first tube 36 introduces it, once heated, newly into the bag 4.

After this preparation stage, the second treatment stage comprises a stage of loading the patient P, a stage of perfusion and a washing stage.

In the loading stage the further clamp 50 is still in a closed configuration and the further clamp 51 is in an open configuration.

The clamp 47 also stays open and the clamp 48 stays closed.

The male connector 42 is disconnected from the female connector 41 and is connected to the inlet P1 of the patient P.

The female connector 41 is connected to the outlet line P2 coming from the patient P. During this loading, the first pump 40 is stationary while the second pump 46 aspirates the liquid solution from the bag 4, sends it to the heating device 43 and then to the patient P.

During this loading stage the abdomen or the pleuric cavity of the patient P is filled, with the greatest possible volume of liquid solution, which is about 4000 ml of liquid solution, while about 1000 ml are left inside the bag 4.

During the following perfusion stage, the configurations of the further clamps 50 and 51 and the clamps 47 and 48 do not change with respect to the loading stage, while only the first pump 40 is activated to aspirate, at the same velocity as the second pump 46, the liquid solution from the abdomen or from the pleuric cavity of the patient P, sending it towards the bag 4 in the direction of arrows R.

During this stage, the solution circulates continuously internally of the set 1, being heated up to the desired temperature, normally 42°C, before being introduced into the patient P and being filtered and collected, as will be more fully described herein below, internally of the medical apparatus 2.

During the circulation, the medical staff can introduce other drugs into the liquid solution to be perfused into the patient P, if this is required.

In order to do this, the operator can fill a syringe with the drug to be introduced and, after having removed the needle, can insert the needle attachment to the inside of the injection point 35 of the introduction hole 13, momentarily opening it towards the receiving element 3, and emptying the drug internally of the receiving chamber 16.

The set 1 is provided with further introduction holes, denoted by 13' and 13", which are exhibited on the first tube 36 and the third tube 45 respectively upstream of the respective pumps 40 and 46.

In detail, during the stage of perfusion the liquid solution exiting from the patient P after having perfused the abdomen or the pleuric cavity thereof is aspirated by the first pump 40 and sent therefrom to the medical apparatus 2.

The liquid solution coming from the patient P is loaded with impurities of various kinds, deriving from the operating field, and is introduced into the receiving chamber 16 through the inlet 5 of the lid 9.

As can be seen in figure 3, the liquid solution, due to the inclination of the bottom 23, tends spontaneously to reach the discharge opening 22 and in order to do this is forced to cross first the filters 19 and 20 and then the filter 21.

Crossing the first filters 19 and 20 eliminates the largest impurities from the liquid solution, while passing through the filter 21, which is more selective, removes the microscopic impurities.

The filtered solution passes through the discharge opening 22 and thence falls into the underlying bag 4, passing through the further filter 27 which slows the speed of fall and prevents the formation of air bubbles or foam.

The filtered liquid solution is then accumulated in the bag 4, from which it is aspirated from the second pump 46 in order to be newly perfused in the patient P.

Worthy of note is the fact that in a case in which the filters 19, 20, 21 were saturated and no longer allowed passage of the liquid solution through the filtering fibres, the emergency passage opening, defined as the difference between height H1 of the separation wall 18 and the filters 19, 20, 21 and height H2 of the box structure 7, i.e. between the separation wall 18 and the lower surface of the lid 9, would enable the filters 19, 20, 21 to be by-passed without interrupting the flow of the liquid solution which would however be subjected to a filtering action on the part of the further filter 27 arranged internally of the bag 4 at the discharge hole 22.

During the following washing stage the further clamp 50 is newly opened while the further clamp 51 is newly closed.

The configuration of the clamps 47 and 48 is unchanged with respect to the configuration of the perfusion stage and the male connector 42 remains connected to the inlet line P1 of the patient P.

The female connector 41 still remains connected to the outlet line P2 from the patient P.

The second pump 46 continues to aspirate the solution from the bag 4 and send it to the heating device 43, and from the heating device to the patient P.

The first pump 40 is active and the functioning regime thereof is modified such that it aspirates the liquid solution which is in the patient P at a greater speed than that with which the liquid solution is sent from the second pump 46 to the patient P.

During this stage, a sort of washing-out of the abdomen or the peritoneal or pleuric cavity of patient P is performed, with the removal of all traces of drug therefrom. During the third stage of emptying, the configurations of the further clamps 50 and 51 and the clamps 47 and 48 does not change.

The male connector 42 still remains connected to the inlet line P1 of the patient P.

The female connector 41 still remains connected to the outlet line P2 from the patient P.

During this stage the second pump 46 is deactivated and only the first pump 40 remains active, which aspirates all the liquid solution from the abdomen or from the peritoneal or pleuric cavity of the patient P and sends it into the bag 4, which has a predetermined capacity sufficient to collect it and contain it entirely.

In this way it is possible to eliminate the whole set 1 according to the existing laws relating to the disposal of materials and device which are in contact with toxic and dangerous drugs, such as, for example, chemotherapy drugs.

## Claims

1. Medical apparatus, particularly suitable for filtration and accumulation of solutions for intra-abdominal and/or intrapleuric perfusion in a hyperthermal regime, comprising:
- a body, said body comprising
- inlet (5) and outlet (6) means for liquid solutions to be filtered;
- filtering means (19, 20, 21) arranged between said inlet (5) and outlet (6) means, said filtering means (19, 20, 21) being arranged in a receiving element (3) of said liquid solutions;
- a container element (4) of said liquid solutions being provided, said container element (4) having a variable capacity and being connected to said receiving element (3) and to said outlet means (6), **characterized in that** between said receiving element (3) and said container element (4) recirculating means are arranged for said liquid solutions which can be connected to a perfusion circuit (1) to a patient (P), between said perfusion circuit (1) and said receiving element (3) in addition to said recirculating means an heating device (43) being provided for and placed along a third tube (45) designed to send the liquid solutions to be perfused to the patient (P) through an inlet (P1) connected to the patient.

2. Apparatus according to claim 1, wherein said receiving element (3) comprises:
- a receiving chamber (16) and a conveying chamber (17) defined separately in said receiving element (3);
- separation wall means (18) arranged in said receiving element (3) to separate said receiving chamber (16) and conveying chamber (17), said conveying chamber (17) being connected to said container element (4).

3. Apparatus according to claim 1 or 2, wherein said wall means (18) form housing seats (24, 25, 26) for arranging said filtering means (19, 20, 21) and permeable towards said receiving chamber (16) and conveying chamber (17), in such a way as to make them communicate.

4. Apparatus according to claim 1, wherein said filtering means comprise filtering elements (19, 20, 21) having at least two different filtration coefficients, first flat filtering elements (19, 20) with low filtration coefficient being fitted in said receiving chamber (16) and arranged in said arrangement seats (24, 25) substantially parallel to said wall means (18) and which can be passed through by said liquid solutions in transversal directions; second filtering elements (21) with high filtration coefficient arranged downstream of said first filtering means (19, 20) turned towards said conveying chamber (17) and comprising at least one mesh filter which comprises a woven no-woven filter (20).

5. Apparatus according to claim 1, wherein said receiving element comprises a box structure (7) which has an upper opening (8) and, on the opposite side, a bottom (23), said upper opening (8) communicating with an external environment and being seal-closable with closing means (9) comprising a third recirculating hole (14) and a fourth communication hole (12) between said external environment and said receiving chamber (16).

6. Apparatus according to anyone of claims 2, 3, 4, wherein said first flat filtering elements (19, 20), said second filtering elements (21) and said wall means (18) have substantially the same height (H1) slightly lower than the height (H2) of said receiving chamber (16) and conveying chamber (17), in such a way as to define a passage opening between said first filtering elements (19, 20), second filtering elements (21), wall means (18) and said closing means (9) when said first filtering means (19, 20) and said second filtering means (21) are obstructed.

7. Apparatus according to claims 1, 2 wherein said closing means comprise:
- a lid (9) that shapes said inlet means (5) at said receiving chamber (16);
- a first compensating hole (11) for connecting said container element (4) with said external environment, in such a way as to compensate pressure differences between said container element (4) and said external environment.

8. Apparatus according to claim 7, wherein said first compensating hole (11) comprises third filtering means that comprise a filter (34) having antibacterial and/or adsorbent filtering elements of molecules in air suspension.

9. Apparatus according to claim 5, wherein said closing means (9) comprise a second introduction hole (15) for introducing additional drugs/substances in said receiving element (3) and which opens into said receiving chamber (16).

10. Apparatus according to any of the claims 1, 7, 9 wherein said inlet means (5), first compensating hole (11), second hole (15), third hole (14) and fourth hole (12) comprise respective mouths extending outside from said closing means (9) and shaping standardized connections known as "Luer connections".

11. Apparatus according to claim 5, wherein said bottom (23) comprises a breather opening (52) that opens inside said box structure (7) and that extends towards the inside of said container element (4).

12. Apparatus according to claim 1, wherein said container element comprises a flexible bag (4) that defines a compartment inside it and that is connected to said box structure (7) on the opposite side to said closing means (9) and below said bottom (23).

13. Apparatus according to claim 11, wherein said bag (4) has further inner inlet tube means (22) which extend into said compartment and having an outlet end opening onto further filtering means (27) arranged in said compartment.

14. Apparatus according to claims 1, 5, 10, wherein said recycling means comprise a duct segment obtained outside said receiving element by said third recirculating hole (14) and shaping a coupling mouth with a duct of a perfusion circuit (1) connected to a patient (P).

## Patentansprüche

1. Medizinische Vorrichtung, insbesondere geeignet zur Filtration und Akkumulation von Lösungen zur intra-abdominalen und/oder intrapleuralen Perfusion in einem hyperthermischen Regime, umfassend:
- einen Körper, wobei der Körper umfasst
- Einlass- (5) und Auslassmittel (6) für zu filtrierende, flüssige Lösungen;
- Filtriermittel (19, 20, 21), angeordnet zwischen dem Einlass- (5) und dem Auslassmittel (6), wobei die Filtriermittel (19, 20, 21) in einem Aufnahmeelement (3) der flüssigen Lösungen angeordnet sind;
- wobei ein Behälterelement (4) der flüssigen Lösungen vorgesehen ist, das Behälterelement (4) ein variables Aufnahmevermögen aufweist und mit dem Aufnahmeelement (3) und dem Auslassmittel (6) verbunden ist, **dadurch gekennzeichnet, dass** zwischen dem Aufnahmeelement (3) und dem Behälterelement (4) Rezirkulierungsmittel für die flüssigen Lösungen angeordnet sind, die an einen Perfusions-Kreis (1) mit einem Patienten (P) verbunden sein können, wobei zwischen dem Perfusions-Kreis (1) und dem Aufnahmeelement (3) zusätzlich zu den Rezirkulierungsmitteln eine Heizvorrichtung (43) vorgesehen und platziert ist, entlang eines dritten Rohrs (45), angelegt, um die zu dem Patienten (P) zu perfundierenden flüssigen Lösungen durch einen mit dem Patienten verbundenen Einlass (P1) zu schicken.

2. Vorrichtung nach Anspruch 1, wobei das Aufnahmeelement (3) umfasst:
- eine Aufnahmekammer (16) und eine Transportkammer (17), die in dem Aufnahmeelement (3) gesondert definiert sind;
- Trennwand-Mittel (18), angeordnet in dem Aufnahmeelement (3) zum Trennen der Aufnahmekammer (16) und Transportkammer (17), wobei die Transportkammer (17) mit dem Behälterelement (4) verbunden ist.

3. Vorrichtung nach Anspruch 1 oder 2, wobei die Wand-Mittel (18) Gehäuseauflagen (24, 25, 26) ausbilden zum Anordnen der Filtriermittel (19, 20, 21) und permeabel zu der Aufnahmekammer (16) und Transportkammer (17) derart, dass sie kommunizieren.

4. Vorrichtung nach Anspruch 1, wobei die Filtriermittel Filtrierelemente (19, 20, 21) mit mindestens zwei verschiedenen Filtrationskoeffizienten umfassen, wobei erste ebene Filtrierelemente (19, 20) mit niedrigem Filtrationskoeffizient in der Aufnahmekammer (16) eingepasst und in den Anordnungsauflagen (24, 25) im Wesentlichen parallel zu den Wand-Mitteln (18) angeordnet sind und durch die die flüssigen Lösungen in Querrichtungen hindurch geführt werden können; wobei zweite Filtrierelemente (21) mit hohem Filtrationskoeffizient stromabwärts der ersten Filtriermittel (19, 20), zu der Transportkammer (17) gedreht, angeordnet sind und mindestens ein Netzfilter, der einen gewebten Vliesfilter (20) umfasst, umfassen.

5. Vorrichtung nach Anspruch 1, wobei das Aufnahmeelement einen KastenAufbau (7) umfasst, der eine obere offene Stelle (8) und an der entgegengesetzten Seite einen Boden (23) aufweist, wobei eine obere offene Stelle (8) mit einer äußeren Umgebung kommuniziert und mit Verschließmitteln (9) abdichtend-verschließbar ist, die eine dritte Rezirkulierungsöffnung (14) und eine vierte Kommunikationsöffnung (12) zwischen der äußeren Umgebung und der Aufnahmekammer (16) umfassen.

6. Vorrichtung nach einem der Ansprüche 2, 3, 4, wobei die ersten ebenen Filtrierelemente (19, 20), die zweiten Filtrierelemente (21) und die Wand-Mittel (18) im Wesentlichen dieselbe Höhe (H1) aufweisen, die etwas niedriger als die Höhe (H2) der Aufnahmekammer (16) und der Transportkammer (17) ist, dergestalt, dass eine durchgangsoffene Stelle zwischen den ersten Filtrierelementen (19, 20), zweiten Filtrierelementen (21), Wand-Mitteln (18) und den Verschließmitteln (9) definiert ist, wenn das erste Filtriermittel (19, 20) und das zweite Filtriermittel (21) verstopft sind.

7. Vorrichtung nach Ansprüchen 1, 2, wobei die Verschließmittel umfassen:
- einen Deckel (9), der das Einlassmittel (5) bei der Aufnahmekammer (16) formt;
- eine erste Ausgleichsöffnung (11) zum Verbinden des Behälterelements (4) mit der äußeren Umgebung, derart dass Druckunterschiede zwischen dem Behälterelement (4) und der äußeren Umgebung ausgeglichen werden.

8. Vorrichtung nach Anspruch 7, wobei die erste Ausgleichsöffnung (11) dritte Filtriermittel umfasst, die einen Filter (34) mit antibakteriellen und/oder von in der Luft schwebenden Molekülen adsorbierenden Filtrierelementen umfassen.

9. Vorrichtung nach Anspruch 5, wobei die Verschließmittel (9) eine zweite Einführungsöffnung (15) zur Einführung zusätzlicher Arzneistoffe/Substanzen in das Aufnahmeelement (3) umfassen und welche in die Aufnahmekammer (16) münden.

10. Vorrichtung nach einem der Ansprüche 1, 7, 9, wobei die Einlassmittel (5), erste Ausgleichsöffnung (11), zweite Öffnung (15), dritte Öffnung (14) und vierte Öffnung (12) jeweilige Mündungen umfassen, die sich außen von den Verschließmitteln (9) erstrecken und standardisierte Verbindungen, die als "Luer-Verbindungen" bekannt sind, formen.

11. Vorrichtung nach Anspruch 5, wobei der Boden (23) eine Entlüftungsöffnung (52) umfasst, die in das Innere des Kastenaufbaus (7) mündet und die sich zum Inneren des Behälterelements (4) erstreckt.

12. Vorrichtung nach Anspruch 1, wobei das Behälterelement einen biegsamen Beutel (4) umfasst, der eine Kammer darin definiert und der mit dem Kastenaufbau (7) auf der entgegengesetzten Seite zu dem Verschließmittel (9) und unter dem Boden (23) verbunden ist.

13. Vorrichtung nach Anspruch 11, wobei der Beutel (4) weitere innere Einlassrohrmittel (22) aufweist, die sich in die Kammer erstrecken und eine Auslassendöffnung auf weiteren Filtriermitteln (27), die in der Kammer angeordnet sind, aufweisen.

14. Vorrichtung nach Ansprüchen 1, 5, 10, wobei die Recyclingmittel ein Leitungssegment umfassen, erhalten außerhalb des Aufnahmeelements durch die dritte Rezirkulierungsöffnung (14) und eine koppelnde Mündung mit einer Leitung eines mit einem Patienten (P) verbundenen Perfusions-Kreises (1) formend.

## Revendications

1. Appareil médical, particulièrement approprié pour la filtration et l'accumulation de solutions pour une perfusion intra-abdominale et/ou intrapleurale dans un régime hyperthermique, comprenant :
- un corps, ledit corps comprenant
- des moyens d'entrée (5) et de sortie (6) pour des solutions liquides à filtrer;
- des moyens de filtration (19, 20, 21) agencés entre lesdits moyens d'entrée (5) et de sortie (6), lesdits moyens de filtration (19, 20, 21) étant agencés dans un élément de réception (3) desdites solutions liquides ;
- un élément (4) faisant office de récipient desdites solutions liquides étant prévu, ledit élément (4) faisant office de récipient possédant une capacité variable et étant relié audit élément de réception (3) et audit moyen de sortie (6), **caractérisé en ce que**, entre ledit élément de réception (3) et ledit élément (4) faisant office de récipient, des moyens de recyclage sont agencés pour lesdites solutions liquides, qui peuvent être reliés à un circuit de perfusion (1) à un patient (P), entre ledit circuit de perfusion (1) et ledit élément de réception (3), en plus desdits moyens de recyclage, un dispositif de chauffage (43) étant prévu et placé le long d'un troisième tube (45) conçu pour envoyer les solutions liquides à perfuser au patient (P) via une entrée (P1) reliée au patient.

2. Appareil selon la revendication 1, dans lequel ledit élément de réception (3) comprend :
- une chambre de réception (16) et une chambre de transport (17) définies séparément dans ledit élément de réception (3) ;
- des moyens (18) faisant office de paroi de séparation agencés dans ledit élément de réception (3) pour séparer ladite chambre de réception (16) et ladite chambre de transport (17), ladite chambre de transport (17) étant reliée audit élément (4) faisant office de récipient.

3. Appareil selon la revendication 1 ou 2, dans lequel lesdits moyens (18) faisant office de paroi forment des sièges de logement (24, 25, 26) destinés à l'agencement desdits moyens de filtration (19, 20, 21) et perméables en direction de ladite chambre de réception (16) et de ladite chambre de transport (17), de manière à les faire communiquer.

4. Appareil selon la revendication 1, dans lequel lesdits moyens de filtration comprennent des éléments de filtration (19, 20, 21) possédant au moins deux coefficients de filtration différents, des premiers éléments de filtration plats (19, 20) possédant un faible coefficient de filtration étant logés dans ladite chambre de réception (16) et agencés dans lesdits sièges d'agencement (24, 25) sensiblement parallèlement auxdits moyens (18) faisant office de paroi et qui peuvent être traversés par lesdites solutions liquides dans des directions transversales; des deuxièmes éléments de filtration (21) possédant un coefficient de filtration élevé, agencés en aval desdits premiers moyens de filtration (19, 20) en étant orientés vers ladite chambre de transport (17) et comprenant au moins un filtre à tamis qui comprend un filtre en tissu non tissé (20).

5. Appareil selon la revendication 1, dans lequel ledit élément de réception comprend une structure (7) en forme de boîte qui possède une ouverture supérieure (8) et, sur le côté opposé, une base (23), ladite ouverture supérieure (8) communiquant avec un environnement externe et pouvant être fermée de manière hermétique avec des moyens de fermeture (9), comprenant un troisième trou de recyclage (14) et un quatrième trou de recyclage (12) entre ledit environnement externe et ladite chambre de réception (16).

6. Appareil selon l'une quelconque des revendications 2, 3, 4, dans lequel lesdits premiers éléments de filtration plats (19, 20), lesdits deuxièmes éléments de filtration (21) et lesdits moyens (18) faisant office de paroi possèdent sensiblement la même hauteur (H1) légèrement inférieure à la hauteur (H2) de ladite chambre de réception (16) et de ladite chambre de transport (17), de façon à définir une ouverture de passage entre lesdits premiers éléments de filtration (19, 20), lesdits deuxièmes éléments de filtration (21), les moyens (18) faisant office de paroi et lesdits moyens de fermeture (9) lorsque lesdits premiers moyens de filtration (19, 20) et lesdits deuxièmes moyens de filtration (21) sont obstrués.

7. Appareil selon les revendications 1, 2, dans lequel lesdits moyens de fermeture comprennent :
- un couvercle (9) qui forme lesdits moyens d'entrée (5) au niveau de ladite chambre de réception (16) ;
- un premier trou de compensation (11) pour relier ledit élément (4) faisant office de récipient audit environnement externe, de façon à compenser des différences de pression entre ledit élément (4) faisant office de récipient et ledit environnement externe.

8. Appareil selon la revendication 7, dans lequel ledit premier trou de compensation (11) comprend des troisièmes moyens de filtration qui comprennent un filtre (34) possédant des éléments de filtration antibactériens et/ou adsorbants, de molécules en suspension dans l'air.

9. Appareil selon la revendication 5, dans lequel lesdits moyens de fermeture (9) comprennent un deuxième trou d'introduction (15) pour introduire des médicaments/substances supplémentaires dans ledit élément de réception (3) et qui s'ouvre dans ladite chambre de réception (16).

10. Appareil selon l'une quelconque des revendications 1, 7, 9, dans lequel lesdits moyens d'entrée (5), le premier trou de compensation (11), le deuxième trou (15), le troisième trou (14) et le quatrième trou (12) comprennent des embouts respectifs s'étendant à l'extérieur desdits moyens de fermeture (9) et prenant la forme de raccords standardisés désignés par le terme « raccords Luer-lock ».

11. Appareil selon la revendication 5, dans lequel ladite base (23) comprend une ouverture d'aération (52) qui s'ouvre à l'intérieur de ladite structure (7) en forme de boîte et qui s'étend en direction de l'intérieur dudit élément (4) faisant office de récipient.

12. Appareil selon la revendication 1, dans lequel ledit élément faisant office de récipient comprend un sac flexible (4) qui définit un compartiment à l'intérieur dudit récipient et qui est relié à ladite structure (7) en forme de boîte du côté opposé auxdits moyens de fermeture (9) et au-dessous de ladite base (23).

13. Appareil selon la revendication 11, dans lequel ledit sac (4) possède des moyens supplémentaires (22) faisant office de tube d'entrée interne, qui s'étendent dans ledit compartiment et possédant une extrémité de sortie s'ouvrant sur des moyens de filtration supplémentaires (27) agencés dans ledit compartiment.

14. Appareil selon les revendications 1, 5, 10, dans lequel lesdits moyens de recyclage comprennent un segment de conduit obtenu à l'extérieur dudit élément de réception par ledit troisième trou de recyclage (14) et formant un embout d'accouplement avec un conduit d'un circuit de perfusion (1) relié à un patient (P).
